# EUROPEAN PATENT APPLICATION

(11) **EP 4 813 301 A1**
(43) Date of publication of application: **30.09.2026**
(21) Application number: 24894188.2
(22) Date of filing: 21.11.2024
(51) Int. Cl.: A61B 6/00, A61B 6/46

(54) **X-RAY IMAGING DEVICE**

(30) Priority: 24.11.2023 JP 2023199415
(71) Applicant: Shimadzu Corporation, Kyoto-shi, Kyoto 604-8511 (JP)
(72) Inventor: NAKATSU, Kohei, Kyoto-shi, Kyoto 604-8511 (JP); FURUHASHI, Naoya, Kyoto-shi, Kyoto 604-8511 (JP); TAKEDA, Ryo, Kyoto-shi, Kyoto 604-8511 (JP); OKUMURA, Hiroshi, Kyoto-shi, Kyoto 604-8511 (JP)
(74) Representative: Müller-Boré & Partner Patentanwälte PartG mbB
(86) International application number: PCT/JP2024/041201
(87) International publication number: WO 2025/110197

(57) **Abstract**

This X-ray imaging apparatus (100) comprises a control unit (32) configured to calculate an X-ray tube-subject distance (D1) between an X-ray tube (11) and a subject (101) based on coordinates of a predetermined position (33c) in an irradiation field region (33a) appearing on a body surface of the subject (101) in an optical image (70) captured by an optical imaging unit (31).

## Description

### Technical Field

The present invention relates to an X-ray imaging apparatus.

### Background Art

Conventionally, an X-ray imaging apparatus including an optical imaging unit is known. Such an X-ray imaging apparatus is disclosed in, for example, Japanese Unexamined Patent Application Publication No. 2012-147978.

Japanese Unexamined Patent Application Publication No. 2012-147978 describes an X-ray imaging apparatus including a camera (optical imaging unit) that is provided in an X-ray tube and captures an image of a subject, and a control unit that recognizes the position of the subject from the image captured by the camera. Prior Art Documents Patent Documents

Patent Document 1: Japanese Unexamined Patent Application Publication No. 2012-147978 Summary of Invention Problems to be Solved by the Invention

Here, when irradiating a subject with X-rays using an X-ray imaging apparatus such as the one in Japanese Unexamined Patent Application Publication No. 2012-147978, it is necessary for an operator (a medical worker such as a radiologic technologist or a doctor) to determine appropriate X-ray conditions (such as the X-ray dose to irradiate the subject) for the subject. Therefore, although not described in Japanese Unexamined Patent Application Publication No. 2012-147978, in order for the operator to determine appropriate X-ray conditions for the subject, in an X-ray imaging apparatus such as the one in Japanese Unexamined Patent Application Publication No. 2012-147978, the control unit designates a point for calculating an X-ray tube-subject distance between the X-ray tube and the subject from a distance value map (an image including distance information) obtained by imaging with a 3D camera, and calculates the X-ray tube-subject distance between the X-ray tube and the subject. In that case, a relatively expensive 3D camera is required. For this reason, a configuration capable of calculating the X-ray tube-subject distance without using a 3D camera is desired.

The present invention has been made to solve the above-described problems, and one object of the present invention is to provide an X-ray imaging apparatus capable of calculating an X-ray tube-subject distance without using a 3D camera. Means for Solving the Problems

An X-ray imaging apparatus according to one aspect of the present invention comprises an X-ray irradiation unit including an X-ray tube; an X-ray detection unit configured to detect X-rays irradiated from the X-ray tube and transmitted through a subject; a light source unit configured to irradiate visible light to an irradiation field region indicating a region to be irradiated with X-rays from the X-ray tube; an optical imaging unit having an optical axis shifted with respect to an irradiation axis of the X-rays irradiated from the X-ray tube, and configured to capture an optical image of the subject; and a control unit configured to calculate an X-ray tube-subject distance between the X-ray tube and the subject based on coordinates of a predetermined position in the irradiation field region of the light source unit appearing on a body surface of the subject in the optical image captured by the optical imaging unit. Effects of the Invention

The X-ray imaging apparatus according to the one aspect includes the control unit configured to calculate the X-ray tube-subject distance between the X-ray tube and the subject based on the coordinates of the predetermined position in the irradiation field region of the light source unit appearing on the body surface of the subject in the optical image captured by the optical imaging unit, as described above. This makes it possible to geometrically calculate the X-ray tube-subject distance based on the coordinates of the predetermined position in the irradiation field region of the light source unit appearing on the body surface of the subject in the optical image. In this case, the optical image only needs to be an image captured by a monocular camera. As a result, the X-ray tube-subject distance can be calculated without using a 3D camera. Brief Description of the Drawings

[FIG. 1] FIG. 1 is a schematic diagram showing an overall configuration of an X-ray imaging apparatus according to a first embodiment.
[FIG. 2] FIG. 2 is a schematic diagram showing a configuration of a holding unit according to the first embodiment.
[FIG. 3] FIG. 3 is a block diagram showing the overall configuration of the X-ray imaging apparatus according to the first embodiment.
[FIG. 4] FIG. 4 is a schematic diagram showing an optical image generated by an optical imaging control unit of the X-ray imaging apparatus according to the first embodiment.
[FIG. 5] FIG. 5 is a schematic diagram for explaining an optical image in which a detection unit region display, an irradiation field region display, and an AEC region display are superimposed on the optical image in the X-ray imaging apparatus according to the first embodiment.
[FIG. 6] FIG. 6 is a schematic diagram (1) for explaining calculation of an X-ray tube-subject distance by the optical imaging control unit of the X-ray imaging apparatus according to the first embodiment.
[FIG. 7] FIG. 7 is a schematic diagram (2) for explaining calculation of the X-ray tube-subject distance by the optical imaging control unit of the X-ray imaging apparatus according to the first embodiment.
[FIG. 8] FIG. 8 is a block diagram showing an overall configuration of an X-ray imaging apparatus according to a second embodiment.
[FIG. 9] FIG. 9 is a schematic diagram (1) for explaining calculation of an X-ray tube-subject distance by an optical imaging control unit of the X-ray imaging apparatus according to the second embodiment.
[FIG. 10] FIG. 10 is a schematic diagram (2) for explaining calculation of the X-ray tube-subject distance by the optical imaging control unit of the X-ray imaging apparatus according to the second embodiment. Mode for Carrying Out the Invention

Hereinafter, embodiments embodying the present invention will be described with reference to the drawings.

### [First Embodiment]

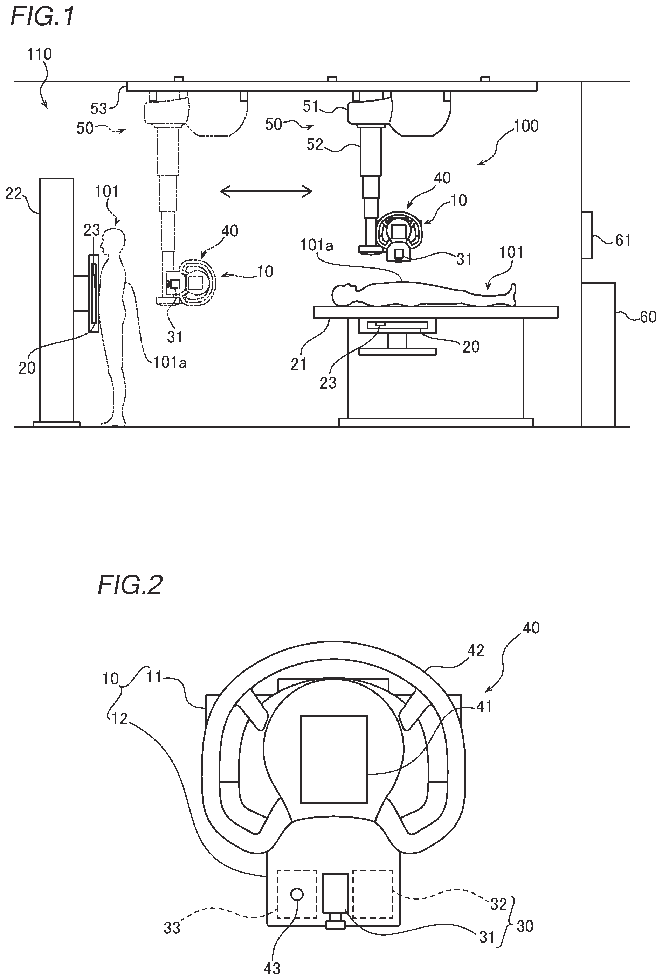

A configuration of an X-ray imaging apparatus 100 according to a first embodiment of the present invention will be described with reference to FIGS. 1 to 7.

### (Overall Configuration of X-ray Imaging Apparatus)

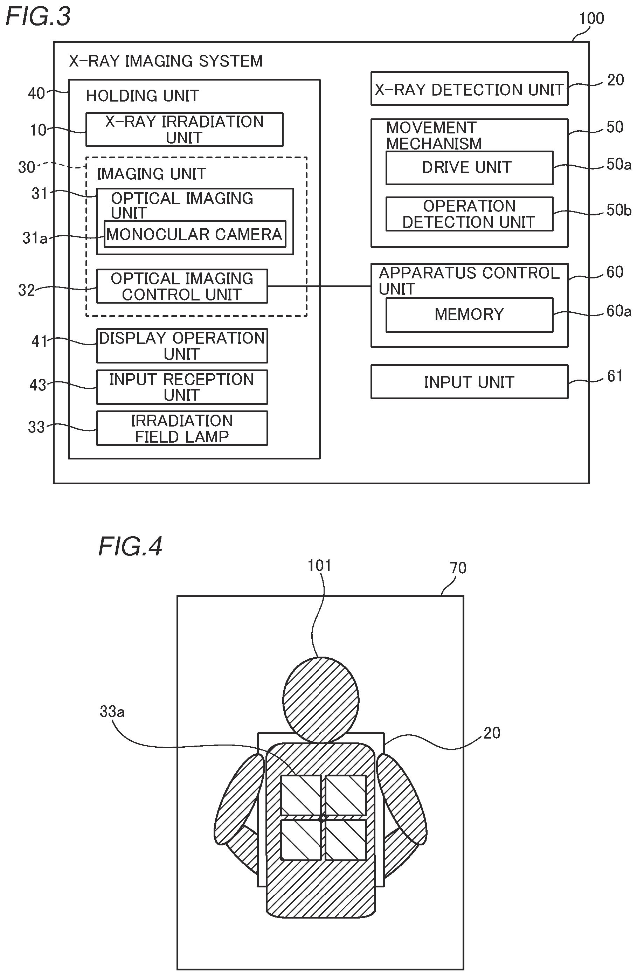

As shown in FIG. 1, the X-ray imaging apparatus 100 includes an X-ray irradiation unit 10, an X-ray detection unit 20, an optical imaging unit 31, a holding unit 40, a movement mechanism 50, an apparatus control unit 60, and an input unit 61. The X-ray imaging apparatus 100 is an imaging apparatus including a medical X-ray imaging apparatus, and is configured to perform X-ray imaging of a subject 101, which is an imaging target. In the X-ray imaging apparatus 100, the X-ray imaging of the subject 101 is performed by the X-ray detection unit 20 detecting X-rays irradiated from the X-ray irradiation unit 10. Further, in the X-ray imaging apparatus 100, an optical image 70 (see FIG. 4) in which the appearance of the subject 101 is imaged is captured by the optical imaging unit 31. Moreover, in the X-ray imaging apparatus 100, the X-ray irradiation unit 10, the X-ray detection unit 20, the optical imaging unit 31, the holding unit 40, and the movement mechanism 50 are installed in an imaging room 110, and the apparatus control unit 60 and the input unit 61 are installed outside the imaging room 110.

The X-ray imaging apparatus 100 is a ceiling-suspended imaging apparatus. In the X-ray imaging apparatus 100, the holding unit 40 holding the X-ray irradiation unit 10 is held so as to be suspended from the ceiling by the movement mechanism 50 arranged on the ceiling of the imaging room 110. The holding unit 40 is movably held within the imaging room 110 by the movement mechanism 50.

The X-ray imaging apparatus 100 includes an imaging table 21 for performing imaging in a posture in which the subject 101 is laid down (decubitus position), and an imaging stand 22 for performing imaging in a posture in which the subject 101 is standing (upright position). The X-ray detection unit 20 is movably held by each of the imaging table 21 and the imaging stand 22. The X-ray detection unit 20 includes, for example, a flat panel detector (FPD). The X-ray detection unit 20 is configured to detect X-rays irradiated from the X-ray irradiation unit 10 and transmitted through the subject 101. The movement mechanism 50 is capable of moving the holding unit 40 at least between an imaging position in the decubitus position using the imaging table 21 (the solid line position in FIG. 1) and an imaging position in the upright position using the imaging stand 22 (the two-dot chain line position in FIG. 1).

The X-ray detection unit 20 is provided with an AEC (Auto Exposure Control) 23. The AEC is a device that detects the X-rays after passing through the subject 101, and controls a tube voltage, a tube current, an irradiation time, and the like of an X-ray tube 11 (see FIG. 2) included in the X-ray irradiation unit 10 according to the detected X-ray dose.

In the X-ray imaging in the decubitus position, the holding unit 40 is arranged at a position vertically facing the X-ray detection unit 20 of the imaging table 21, and X-ray imaging is performed on the subject 101 lying on the imaging table 21 between the vertically facing X-ray irradiation unit 10 and the X-ray detection unit 20, and the subject 101 lying on the imaging table 21 is imaged (optically captured) by the optical imaging unit 31. In the X-ray imaging in the upright position, the holding unit 40 is arranged at a position horizontally facing the X-ray detection unit 20 of the imaging stand 22, and X-ray imaging is performed on the subject 101 standing in front of the imaging stand 22 between the horizontally facing X-ray irradiation unit 10 and the X-ray detection unit 20, and the subject 101 standing in front of the imaging stand 22 is imaged by the optical imaging unit 31.

The movement mechanism 50 is configured to hold the holding unit 40 so as to be movable in the horizontal direction and the vertical direction. The movement mechanism 50 includes a ceiling suspender 51 and a column unit 52. The movement mechanism 50 is supported by rails 53 provided on the ceiling of the imaging room 110. The ceiling suspender 51 is configured to be movable in the horizontal direction by the rails 53. The ceiling suspender 51 is configured to support the column unit 52. The column unit 52 is configured to support the holding unit 40. The column unit 52 is configured to be extendable and contractible in the vertical direction. The holding unit 40 is configured to be movable in the vertical direction by the column unit 52. Further, the movement mechanism 50 moves the X-ray detection unit 20 arranged in each of the imaging table 21 and the imaging stand 22.

As shown in FIG. 2, the X-ray irradiation unit 10 includes the X-ray tube 11 and a collimator unit 12. The X-ray irradiation unit 10 is configured to irradiate X-rays from the X-ray tube 11 onto the subject 101 (see FIG. 1). The X-ray tube 11 is configured to irradiate X-rays by applying a predetermined voltage. The collimator unit 12 has a plurality of position-adjustable shielding plates (collimator leaves). The collimator unit 12 is configured to adjust the irradiation field of the X-rays irradiated from the X-ray tube 11 by shielding a part of the X-rays from the X-ray tube 11. The collimator unit 12 is provided in the vicinity of the X-ray tube 11 in the X-ray irradiation direction of the X-ray tube 11.

The holding unit 40 includes a display operation unit 41 and a grip unit 42. The holding unit 40 is configured to be movable in the horizontal direction and the vertical direction via the movement mechanism 50 (see FIG. 1) by manual movement or control by the apparatus control unit 60 (see FIG. 1). The optical imaging unit 31 is provided in the holding unit 40 together with the X-ray irradiation unit 10. Specifically, an imaging unit 30 composed of the optical imaging unit 31 and an optical imaging control unit 32 is arranged in the holding unit 40. Further, an irradiation field lamp 33 and an input reception unit 43 are also provided in the holding unit 40. The optical imaging control unit 32, the display operation unit 41, and the irradiation field lamp 33 are examples of a "control unit", a "display unit", and a "light source unit" in the claims, respectively.

The display operation unit 41 includes, for example, a touch panel type liquid crystal display. The display operation unit 41 is configured to function as an image display unit that displays the optical image 70 (see FIG. 4) captured by the optical imaging unit 31 and the like, and also function as an operation unit into which various operations by an operator (a medical worker such as a radiologic technologist or a doctor) are input. The display operation unit 41 outputs a signal indicating an accepted input operation to the optical imaging control unit 32 and the apparatus control unit 60 (see FIG. 1).

The grip unit 42 is provided for the operator to grip when performing an operation to manually move the holding unit 40. The grip unit 42 transmits an operation force of the operator to the holding unit 40.

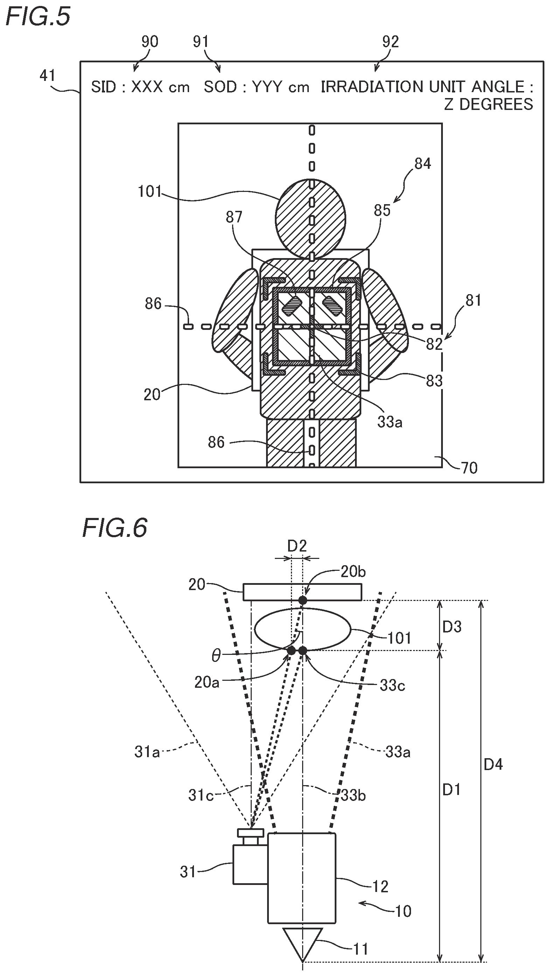

The optical imaging unit 31 is provided on an outer side surface of the collimator unit 12. The optical imaging unit 31 is provided on an outer side surface on a longitudinal side of the imaging table 21 (see FIG. 1) in the collimator unit 12 at the imaging position in the decubitus position. Further, the optical imaging unit 31 is provided on an outer side surface on a side surface side crossing the detection surface of the X-ray detection unit 20 (see FIG. 1) in the collimator unit 12 at the imaging position in the upright position. The optical imaging unit 31 is provided facing the X-ray irradiation direction from the X-ray tube 11. The optical imaging unit 31 is capable of capturing the optical image 70 (see FIG. 4) of the subject 101 and the X-ray detection unit 20 from the X-ray irradiation unit 10 side when the X-ray irradiation unit 10 faces the subject 101 (see FIG. 1) and the X-ray detection unit 20. An imaging range 31a (see FIG. 6) of the optical imaging unit 31 is set to include the range of the irradiation field to which X-rays are irradiated, and to be larger than the range of the irradiation field of the X-rays.

The irradiation field lamp 33 and the input reception unit 43 are provided in the collimator unit 12. The irradiation field lamp 33 is provided inside the collimator unit 12. The irradiation field lamp 33 is configured to irradiate visible light onto an irradiation field region 33a (see FIG. 6) indicating a region to be irradiated with X-rays by the X-ray irradiation unit 10. Specifically, a part of the visible light irradiated from the irradiation field lamp 33 is shielded by a plurality of shielding plates (collimator leaves) of the collimator unit 12, similarly to the X-rays irradiated from the X-ray irradiation unit 10, whereby the irradiation field is adjusted. Therefore, the irradiation field of the visible light irradiated from the irradiation field lamp 33 becomes equal to the irradiation field of the X-rays irradiated from the X-ray irradiation unit 10 by being adjusted by the plurality of shielding plates (collimator leaves) of the collimator unit 12. An optical axis 33b (see FIG. 6) of the irradiation field lamp 33 coincides with an optical axis of the X-rays irradiated from the X-ray irradiation unit 10. The irradiation field lamp 33 includes a light emitting element such as an LED (Light Emitting Diode), for example.

The input reception unit 43 is configured to receive an input of an operator. The input reception unit 43 is, for example, a push button for operating the irradiation field lamp 33. That is, the input reception unit 43 is configured to receive an operation input for turning on the irradiation field lamp 33. When the input reception unit 43 is operated (pressed) by the operator, visible light is irradiated from the irradiation field lamp 33.

As shown in FIG. 3, the optical imaging unit 31 includes a monocular camera 31b (2D camera). On the other hand, the optical imaging unit 31 does not include a 3D camera. The monocular camera 31b includes an image sensor such as a CCD (Charge Coupled Device) image sensor or a CMOS (Complementary Metal Oxide Semiconductor) image sensor, for example.

The optical imaging control unit 32 is a microcomputer including a CPU (Central Processing Unit) and a memory, for example. The optical imaging control unit 32 transmits and receives signals to and from the apparatus control unit 60 by a wireless connection or a wired connection.

As shown in FIG. 4, the optical imaging unit 31 (see FIG. 3) is configured to capture the optical image 70 of the subject 101. The optical imaging control unit 32 (see FIG. 3) receives an input of a signal output from the optical imaging unit 31, and is configured to generate the optical image 70 based on the input signal. Furthermore, the optical imaging control unit 32 is configured to perform control to output the generated optical image 70 to the display operation unit 41 (see FIG. 3) and display it. FIG. 4 shows the optical image 70 in a state where visible light irradiated from the irradiation field lamp 33 (see FIG. 3) is irradiated to the irradiation field region 33a (see FIG. 6). Therefore, the optical image 70 shown in FIG. 4 captures the subject 101, the X-ray detection unit 20, and the irradiation field region 33a. Note that when the visible light is not irradiated from the irradiation field lamp 33, the irradiation field region 33a is not captured in the optical image 70.

As shown in FIG. 3, the apparatus control unit 60 includes a CPU and a memory 60a. The apparatus control unit 60 controls X-ray imaging by the X-ray irradiation unit 10 and the X-ray detection unit 20, and controls movement of the holding unit 40. The input unit 61 has a function of receiving an input operation related to X-ray imaging. The input operations include setting of imaging conditions for X-ray imaging, instructions to start X-ray irradiation, and the like. The apparatus control unit 60 performs control of X-ray imaging based on parameters and various programs set in advance and stored in the memory 60a. The apparatus control unit 60 controls an adjustment amount of the irradiation field by the collimator unit 12 (see FIG. 2).

The movement mechanism 50 includes a drive unit 50a such as a motor, and an operation detection unit 50b that detects the operation of the drive unit 50a. The operation detection unit 50b includes, for example, a potentiometer that detects the rotation of the motor. The drive unit 50a and the operation detection unit 50b are arranged in each unit of the movement mechanism 50 for moving the holding unit 40, and are also arranged in each of the imaging table 21 (see FIG. 1) and the imaging stand 22 (see FIG. 1) to change the position of the X-ray detection unit 20. The apparatus control unit 60 changes the position and angle of the X-ray irradiation unit 10 and the position of the X-ray detection unit 20 by controlling the operation of the movement mechanism 50 based on an input operation to the input unit 61 (see FIG. 1) or the display operation unit 41, or an operation force applied to the grip unit 42 (see FIG. 2) of the holding unit 40. Further, the apparatus control unit 60 controls the operation of the movement mechanism 50 by feedback control by outputting a control signal for controlling the operation of the drive unit 50a and receiving a detection signal indicating the operation of the drive unit 50a detected by the operation detection unit 50b.

The apparatus control unit 60 outputs the position and angle of the X-ray irradiation unit 10 held by the holding unit 40, and the position of the X-ray detection unit 20 arranged in each of the imaging table 21 (see FIG. 1) and the imaging stand 22 (see FIG. 1) to the optical imaging control unit 32. The optical imaging control unit 32 acquires the arrangement of the X-ray irradiation unit 10 and the arrangement of the X-ray detection unit 20 based on the input from the apparatus control unit 60. Further, the optical imaging control unit 32 acquires an adjustment amount of the irradiation field by the collimator unit 12 based on the input from the apparatus control unit 60.

### (Control of Display by Optical Imaging Control Unit)

As shown in FIG. 5, the optical imaging control unit 32 (see FIG. 3) is configured to display a detection unit region display 81, an irradiation field region display 84, and an AEC region display 87 superimposed on the optical image 70 on the display operation unit 41. The optical imaging control unit 32 causes the display operation unit 41 to display the detection unit region display 81 based on the calculated X-ray tube-subject distance D1 (see FIG. 6). Details of the calculation of the X-ray tube-subject distance D1 will be described later. In the following description, the display control of the display operation unit 41 by the optical imaging control unit 32 when performing X-ray imaging in a posture in which the subject 101 is standing (upright position) will be described. Further, since the above control when performing X-ray imaging in a posture in which the subject 101 is laid on the imaging table 21 (see FIG. 1) (decubitus position) is also the same control, description thereof is omitted.

The detection unit region display 81 indicates an area where the X-ray detection unit 20 (see FIG. 3) is arranged in the optical image 70. The optical imaging control unit 32 (see FIG. 3) acquires the three-dimensional arrangement of the X-ray irradiation unit 10 (see FIG. 3) and the X-ray detection unit 20, and acquires the three-dimensional arrangement of the optical imaging unit 31 (see FIG. 3). Since the optical imaging unit 31 is arranged in the holding unit 40 (see FIG. 3) together with the X-ray irradiation unit 10, the optical imaging control unit 32 calculates a three-dimensional position of the optical imaging unit 31 using parameters stored in advance, based on the acquired position of the X-ray irradiation unit 10, for example. Then, based on the three-dimensional positional relationship among the acquired X-ray irradiation unit 10, the X-ray detection unit 20, and the optical imaging unit 31, the optical imaging control unit 32 performs a geometric calculation to detect an area where the X-ray detection unit 20 is arranged in the optical image 70. The optical imaging control unit 32 may acquire the position of the optical imaging unit 31 calculated by the apparatus control unit 60 (see FIG. 3).

The optical imaging control unit 32 (see FIG. 3) generates the detection unit region display 81 based on the detected region where the X-ray detection unit 20 (see FIG. 3) is arranged. The detection unit region display 81 shows the region where the X-ray detection unit 20 is arranged in a rectangular shape. The detection unit region display 81 indicates a rectangular area, for example. The detection unit region display 81 has a center display 82 indicating the center of the region where the X-ray detection unit 20 is arranged, and four region displays 83 indicating the four corners of a rectangular region by an L-shape. The center display 82 has a cross shape, and is arranged at a position where diagonal lines of the rectangular region intersect. The center display 82 and the four region displays 83 are colored and displayed in blue, for example. In addition, the detection unit region display 81 indicates a guide for a position and a range of an area where X-rays can be detected by the actual X-ray detection unit 20 in the optical image 70. The area indicated by the detection unit region display 81 may be a range smaller than the area where X-rays can be detected by the actual X-ray detection unit 20, or may be a larger range.

Further, the optical imaging control unit 32 (see FIG. 3) is configured to perform control to superimpose an irradiation field region display 84 indicating the irradiation field region 33a (see FIG. 6) together with the detection unit region display 81 onto the optical image 70 and display it on the display operation unit 41. Similarly to the detection unit region display 81, the optical imaging control unit 32 detects an irradiation field region 33a irradiated with X-rays by the X-ray irradiation unit 10 in the optical image 70 by performing a geometric calculation based on the acquired three-dimensional positional relationship among the X-ray irradiation unit 10 (see FIG. 3), the X-ray detection unit 20 (see FIG. 3), and the optical imaging unit 31 (see FIG. 3). The optical imaging control unit 32 then generates the irradiation field region display 84 based on the detected irradiation field region 33a irradiated with X-rays.

The irradiation field region display 84 shows an area to be irradiated with X-rays in a rectangular shape. The irradiation field region display 84 shows a square area, for example. The irradiation field region display 84 has a region display 85 which is a rectangular frame line showing the irradiation field region 33a (see FIG. 6) irradiated with X-rays, and a pair of linear dotted-line displays 86 intersecting with each other at right angles so as to indicate the center of the irradiation field region 33a irradiated with X-rays. The pair of dotted-line displays 86 are arranged while mutually intersecting at right angles so as to pass through a center position where the diagonal lines of the rectangle of the region display 85 intersect. Further, the pair of dotted-line displays 86 are arranged so as to extend outward from the region display 85 which is a rectangular frame line. The region display 85 and the pair of dotted-line displays 86 are colored and displayed in a different color (for example, yellow) from the detection unit region display 81. In addition, the irradiation field region display 84 shows a guide for the position and range of the region irradiated with X-rays in the optical image 70. The region indicated by the irradiation field region display 84 may be a range smaller than the region actually irradiated with X-rays, or may be a larger range.

The optical imaging control unit 32 (see FIG. 3) is configured to change the size of the irradiation field region display 84 superimposed on the optical image 70 in the display operation unit 41 (see FIG. 3) according to an X-ray irradiation field defined by adjusting the plurality of shielding plates of the collimator unit 12 (see FIG. 2). The optical imaging control unit 32 changes the size of the region display 85 which is a rectangular frame line in the irradiation field region display 84 superimposed on the optical image 70, based on values of adjustment amounts of the plurality of shielding plates of the collimator unit 12 acquired from the apparatus control unit 60 (see FIG. 3). Note that even if the size of the region display 85 indicated by the rectangular frame line is changed in the irradiation field region display 84, the size of the dotted-line displays 86 is not changed.

The AEC region display 87 indicates the position and shape of the AEC 23 (see FIG. 1) provided in the X-ray detection unit 20 (see FIG. 3). The position and shape where the AEC 23 is provided in the X-ray detection unit 20 are known. Furthermore, the optical imaging control unit 32 (see FIG. 3) detects a region where the X-ray detection unit 20 is arranged in the optical image 70 when generating the detection unit region display 81, as described above. Therefore, the optical imaging control unit 32 generates the AEC region display 87 based on the detected arrangement of the X-ray detection unit 20 and the position and shape of the AEC 23 in the X-ray detection unit 20. The AEC is provided in the X-ray detection unit 20. Thus, the AEC region display 87 is colored and displayed in the same color (for example, blue) as the detection unit region display 81. In the example shown in FIG. 5, two AEC region displays 87 are superimposed on the optical image 70.

### (Calculation of X-ray Tube-Subject Distance)

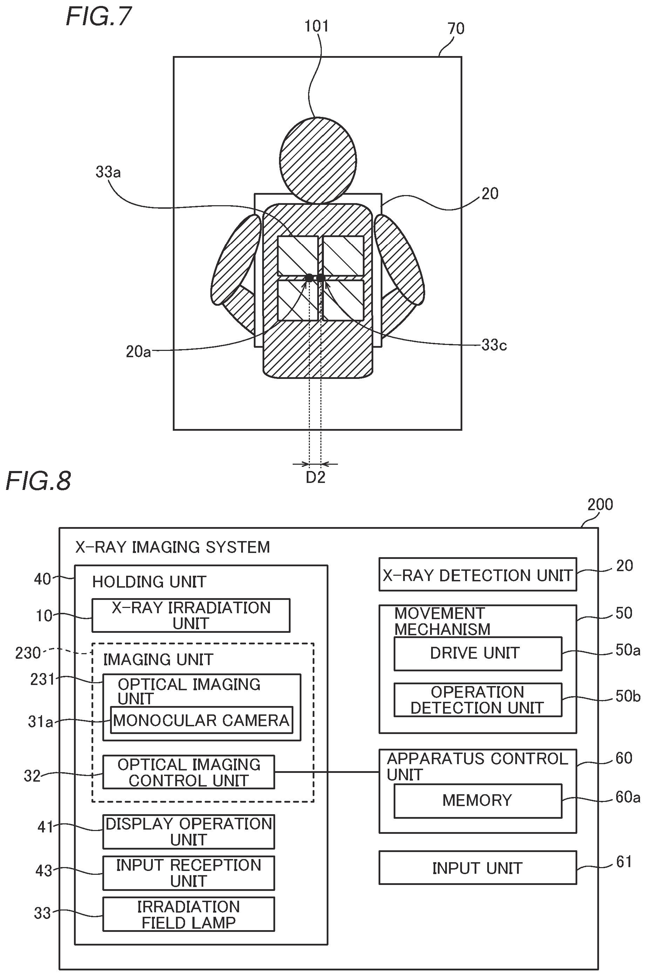

Here, when irradiating the subject 101 with X-rays using the X-ray imaging apparatus 100 (see FIG. 1), an operator (a medical worker such as a radiologic technologist or a doctor) needs to determine appropriate X-ray conditions (such as an X-ray dose to irradiate the subject 101) for the subject 101. Therefore, in order for the operator to determine appropriate X-ray conditions for the subject 101, as shown in FIGS. 6 and 7, the optical imaging control unit 32 (see FIG. 3) calculates an X-ray tube-subject distance D1 between the X-ray tube 11 (focal point thereof) and the subject 101 based on coordinates of a center as a predetermined position 33c in the irradiation field region 33a appearing on the body surface of the subject 101 in the optical image 70 captured by the monocular camera 31b (see FIG. 3) of the optical imaging unit 31.

Specifically, the optical imaging control unit 32 (see FIG. 3) calculates a detection unit-body surface distance D3 between the X-ray detection unit 20 and the body surface of the subject 101 in the optical axis direction of the irradiation field lamp 33 (see FIG. 3), based on a coordinate difference D2 between coordinates of a center as a predetermined position 20a of the X-ray detection unit 20 in the optical image 70 and coordinates of a center as a predetermined position 33c in the irradiation field region 33a appearing on the body surface of the subject 101 in the optical image 70. Then, the optical imaging control unit 32 calculates the X-ray tube-subject distance D1 based on the calculated detection unit-body surface distance D3 and an X-ray tube-detection unit distance D4 between the X-ray tube 11 and the X-ray detection unit 20.

In detail, as shown in FIG. 6, an optical axis 31c of the optical imaging unit 31 is parallel to the optical axis 33b of the irradiation field lamp 33. In other words, the optical imaging unit 31 has an optical axis shifted in a direction in which the optical imaging unit 31 and the X-ray irradiation unit 10 are aligned, with respect to the irradiation axis of the X-rays irradiated from the X-ray irradiation unit 10 (the optical axis 33b of the irradiation field lamp 33). For this reason, as shown in FIG. 7, in the optical image 70, the center as the predetermined position 20a of the X-ray detection unit 20 and the center as the predetermined position 33c in the irradiation field region 33a appearing on the body surface of the subject 101 are shifted from each other. Therefore, the optical imaging control unit 32 (see FIG. 3) first calculates a coordinate difference D2 between coordinates of the center as the predetermined position 20a of the X-ray detection unit 20 in the optical image 70 and coordinates of the center as the predetermined position 33c in the irradiation field region 33a appearing on the body surface of the subject 101 in the optical image 70.

Next, as shown in FIG. 6, the optical imaging control unit 32 (see FIG. 3) calculates the detection unit-body surface distance D3 between the X-ray tube 11 and the X-ray detection unit 20 from an equation: tan θ = (coordinate difference D2 / detection unit-body surface distance D3), where θ is an angle formed by the optical axis 33b of the irradiation field lamp 33 and a straight line connecting the optical imaging unit 31 and a center 20b of the X-ray detection unit 20. That is, the optical imaging control unit 32 calculates the detection unit-body surface distance D3 in the optical axis direction of the X-ray tube 11 based on the angle θ formed by the optical axis 33b of the irradiation field lamp 33 and the straight line connecting the optical imaging unit 31 and the center 20b of the X-ray detection unit 20, in addition to the coordinate difference D2. Next, the optical imaging control unit 32 subtracts the detection unit-body surface distance D3 from the X-ray tube-detection unit distance D4 to calculate the X-ray tube-subject distance D1.

As shown in FIG. 5, the optical imaging control unit 32 (see FIG. 3) displays the calculated X-ray tube-subject distance D1 (see FIG. 6) on the display operation unit 41. Specifically, the optical imaging control unit 32 displays a display region 91 for displaying the X-ray tube-subject distance D1 on the display operation unit 41. Furthermore, in addition to the X-ray tube-subject distance D1, the optical imaging control unit 32 displays, on the display operation unit 41, an SID (Source to Image receptor Distance) which is a distance between the X-ray tube 11 (see FIG. 2) (focal point thereof) and the X-ray detection unit 20 (see FIG. 1), an irradiation unit angle representing an angle of the X-ray irradiation unit 10 (see FIG. 1) relative to the imaging table 21 (see FIG. 1) or the imaging stand 22 (see FIG. 1), and the like. Specifically, the optical imaging control unit 32 displays a display region 90 displaying the SID, a display region 92 displaying the irradiation unit angle, and the like on the display operation unit 41. In FIG. 5, the X-ray tube-subject distance D1 is displayed as SOD.

### (Effects of First Embodiment)

In the first embodiment, the following effects can be obtained.

In the first embodiment, as described above, the X-ray imaging apparatus 100 includes the optical imaging control unit 32 (control unit) configured to calculate the X-ray tube-subject distance D1 between the X-ray tube 11 and the subject 101 based on coordinates of the predetermined position 33c in the irradiation field region 33a appearing on the body surface of the subject 101 in the optical image 70 captured by the optical imaging unit 31. This makes it possible to geometrically calculate the X-ray tube-subject distance D1 based on the coordinates of the predetermined position 33c in the irradiation field region 33a appearing on the body surface of the subject 101 in the optical image 70. In this case, the optical image 70 only needs to be an image captured by the monocular camera 31b. As a result, the X-ray tube-subject distance D1 can be calculated without using a 3D camera.

Further, in the first embodiment, by configuring as described below, further effects as below are obtained.

That is, in the first embodiment, as described above, the optical imaging unit 31 includes the monocular camera 31b. Then, the optical imaging control unit 32 (control unit) calculates the X-ray tube-subject distance D1 based on coordinates of the predetermined position 33c in the irradiation field region 33a appearing on the body surface of the subject 101 in the optical image 70 captured by the monocular camera 31b. With such a configuration, a configuration capable of calculating the X-ray tube-subject distance D1 without using a 3D camera can be reliably realized.

Further, in the first embodiment, as described above, the optical imaging control unit 32 (control unit) calculates the detection unit-body surface distance D3 between the X-ray detection unit 20 and the body surface of the subject 101 in the optical axis direction of the X-ray tube 11 based on the coordinate difference D2 between coordinates of the predetermined position 20a of the X-ray detection unit 20 in the optical image 70 and coordinates of the predetermined position 33c in the irradiation field region 33a appearing on the body surface of the subject 101 in the optical image 70. Then, the optical imaging control unit 32 calculates the X-ray tube-subject distance D1 based on the calculated detection unit-body surface distance D3 and the X-ray tube-detection unit distance D4 between the X-ray tube 11 and the X-ray detection unit 20. With such a configuration, it is possible to geometrically calculate the detection unit-body surface distance D3 based on the coordinate difference D2 and the coordinates of the predetermined position 20a of the X-ray detection unit 20 in the optical image 70, which is information recognizable by the optical imaging control unit 32, such as relative positional relationships of respective parts of the X-ray imaging apparatus 100, and also possible to calculate the X-ray tube-subject distance D1 by subtracting the detection unit-body surface distance D3 from the X-ray tube-detection unit distance D4.

Further, in the first embodiment, as described above, the optical imaging control unit 32 (control unit) calculates the detection unit-body surface distance D3 in the optical axis direction of the irradiation field lamp 33 based on the angle θ formed by the optical axis of the irradiation field lamp 33 (light source unit) and the straight line connecting the optical imaging unit 31 and the center 20b of the X-ray detection unit 20, in addition to the coordinate difference D2. With such a configuration, the detection unit-body surface distance D3 can be easily and geometrically calculated by the coordinate difference D2 and the angle θ using a geometric relationship of tan θ = (coordinate difference D2 / detection unit-body surface distance D3).

Further, in the first embodiment, as described above, the optical imaging control unit 32 (control unit) calculates the X-ray tube-subject distance D1 based on coordinates of a center as the predetermined position 33c in the irradiation field region 33a appearing on the body surface of the subject 101 in the optical image 70. With such a configuration, by using a mark indicating the center of the irradiation field region 33a generally displayed at the center of the irradiation field region 33a in the optical image 70, coordinates of the center as the predetermined position 33c of the irradiation field region 33a in the optical image 70 can be easily determined. As a result, the X-ray tube-subject distance D1 can be easily calculated, as compared to a case of using coordinates other than the center as the coordinates of the predetermined position 33c among the irradiation field region 33a appearing on the body surface of the subject 101 in the optical image 70.

Further, in the first embodiment, as described above, the optical axis of the optical imaging unit 31 is parallel to the optical axis of the irradiation field lamp 33 (light source unit). With such a configuration, when calculating the X-ray tube-subject distance D1, it is not necessary to consider that the optical axis of the optical imaging unit 31 is inclined with respect to the optical axis of the irradiation field lamp 33, so that the X-ray tube-subject distance D1 can be easily calculated, as compared to a case where the optical axis of the optical imaging unit 31 is not parallel to the optical axis of the irradiation field lamp 33.

Further, in the present embodiment, as described above, the X-ray imaging apparatus 100 includes the display operation unit 41 (display unit). Then, the optical imaging control unit 32 (control unit) causes the display operation unit 41 to display the calculated X-ray tube-subject distance D1. With such a configuration, the operator can easily and visually recognize the X-ray tube-subject distance D1 displayed on the display operation unit 41. As a result, the operator can easily determine appropriate X-ray conditions for the subject.

Further, in the present embodiment, as described above, the optical imaging control unit 32 (control unit) causes the display operation unit 41 (display unit) to display the detection unit region display 81 indicating a region where the X-ray detection unit 20 is arranged in the optical image 70, based on the calculated X-ray tube-subject distance D1. With such a configuration, the operator can easily and visually recognize the detection unit region display 81 displayed on the display operation unit 41. As a result, the operator can easily grasp the position where the X-ray detection unit 20 exists.

### [Second Embodiment]

A configuration of an X-ray imaging apparatus 200 according to a second embodiment of the present invention will be described with reference to FIGS. 8 to 10. In the figures, the same portions as those in the first embodiment are assigned the same reference numerals.

### (Overall Configuration of X-ray Imaging Apparatus)

As shown in FIG. 8, the X-ray imaging apparatus 200 includes a holding unit 40. In the holding unit 40, an imaging unit 230 composed of an optical imaging unit 31 and an optical imaging control unit 232 is arranged. The optical imaging control unit 232 is an example of a "control unit" in the claims.

### (Calculation of X-ray Tube-Subject Distance)

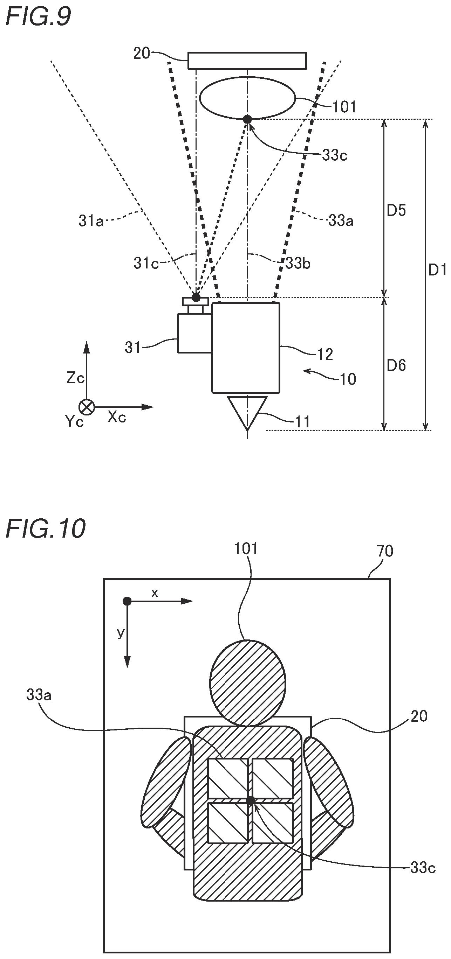

As shown in FIGS. 9 and 10, similarly to the first embodiment, the optical imaging control unit 232 (see FIG. 8) calculates an X-ray tube-subject distance D1 between the X-ray tube 11 and the subject 101 based on coordinates of a center as a predetermined position 33c in an irradiation field region 33a appearing on the body surface of the subject 101 in an optical image 70 captured by a monocular camera 31b (see FIG. 8) of the optical imaging unit 31 (see FIG. 8).

Specifically, the optical imaging control unit 232 (see FIG. 8) calculates an imaging unit-body surface distance D5 between the optical imaging unit 31 and the body surface of the subject 101 in the optical axis direction of an irradiation field lamp 33 (see FIG. 8), based on coordinates of the center as the predetermined position 33c in the irradiation field region 33a appearing on the body surface of the subject 101 in the optical image 70, coordinates of an imaging unit coordinate system centered on the optical imaging unit 31, and a relational expression for converting coordinates of the imaging unit coordinate system into coordinates of an image coordinate system indicating coordinates in the optical image 70 (a relational expression using a matrix of internal parameters of the optical imaging unit 31). Then, the optical imaging control unit 232 calculates the X-ray tube-subject distance D1 based on the calculated imaging unit-body surface distance D5 and an X-ray tube-imaging unit distance D6 between the X-ray tube 11 and the optical imaging unit 31 in the optical axis direction of the irradiation field lamp 33.

In detail, assuming an irradiation axis direction of the X-ray irradiation unit 10 as a Z direction, and mutually orthogonal directions within a plane orthogonal to the Z direction as an X direction and a Y direction, respectively, and assuming coordinates of the imaging unit coordinate system (Xc, Yc, Zc) in real space centered (origin) on the optical imaging unit 31 for coordinates of the center as the predetermined position 33c in the irradiation field region 33a appearing on the body surface of the subject 101 in the optical image 70, and coordinates of the image coordinate system (x, y) indicating coordinates in the optical image 70, to be (Xcₕ, Ycₕ, Zcₕ) and (xₕ, yₕ), respectively, the following equation (1) is established.
[Equation 1] $\left[\begin{matrix}{x}_{h} \\ {y}_{h} \\ 1\end{matrix}\right] = K \left[\begin{matrix}{Xc}_{h} / {Zc}_{h} \\ {Yc}_{h} / {Zc}_{h} \\ 1\end{matrix}\right] = \left[\begin{matrix}\frac{w}{Sx} \times f & 0 & w / 2 \\ 0 & \frac{h}{Sy} \times f & h / 2 \\ 0 & 0 & 1\end{matrix}\right] \left[\begin{matrix}{Xc}_{h} / {Zc}_{h} \\ {Yc}_{h} / {Zc}_{h} \\ 1\end{matrix}\right]$ Here, K is a matrix for converting coordinates of the imaging unit coordinate system into coordinates of the image coordinate system, and is what is called a matrix of internal parameters of the optical imaging unit 31. That is, f is a focal length of a lens of the monocular camera 31b of the optical imaging unit 31, (Sx, Sy) is a size of an image sensor of the monocular camera 31b of the optical imaging unit 31, and (w, h) is the number of pixels of the image sensor of the monocular camera 31b of the optical imaging unit 31.

From the above equation (1), the following equations (2) and (3) are derived.
[Equation 2] ${x}_{h} = \frac{w}{Sx} \times f \times \frac{{Xc}_{h}}{{Zc}_{h}} + \frac{w}{2}$ [Equation 3] ${y}_{h} = \frac{h}{Sy} \times f \times \frac{{Yc}_{h}}{{Zc}_{h}} + \frac{h}{2}$

From the above equation (2) and equation (3), the following equations (4) and (5) are respectively derived.
[Equation 4] ${Zc}_{h} = \frac{\frac{w}{Sx} \times f \times {Xc}_{h}}{{x}_{h} - \frac{w}{2}}$ [Equation 5] ${Zc}_{h} = \frac{\frac{h}{Sy} \times f \times {Yc}_{h}}{{y}_{h} - \frac{h}{2}}$

Thereby, Zcₕ can be calculated from the above equation (4) or equation (5). Zcₕ is a Z direction component of coordinates of the imaging unit coordinate system centered (origin) on the optical imaging unit 31 for coordinates of the center as the predetermined position 33c in the irradiation field region 33a appearing on the body surface of the subject 101 in the optical image 70. Zcₕ is equal to the imaging unit-body surface distance D5.

Then, the optical imaging control unit 232 (see FIG. 8) adds the imaging unit-body surface distance D5 and the X-ray tube-imaging unit distance D6 between the X-ray tube 11 and the optical imaging unit 31 in the optical axis direction of the irradiation field lamp 33 (see FIG. 8) to calculate the X-ray tube-subject distance D1.

Note that other configurations of the second embodiment are the same as those of the first embodiment.

### (Effects of Second Embodiment)

In the second embodiment, the following effects can be obtained.

In the second embodiment, as described above, the X-ray imaging apparatus 200 includes the optical imaging control unit 232 (control unit) configured to calculate the X-ray tube-subject distance D1 between the X-ray tube 11 and the subject 101 based on coordinates of the predetermined position 33c in the irradiation field region 33a appearing on the body surface of the subject 101 in the optical image 70 captured by the optical imaging unit 31. This makes it possible to geometrically calculate the X-ray tube-subject distance D1 based on the coordinates of the predetermined position 33c in the irradiation field region 33a appearing on the body surface of the subject 101 in the optical image 70. In this case, similarly to the first embodiment, the optical image 70 only needs to be an image captured by the monocular camera 31b. As a result, similarly to the first embodiment, the X-ray tube-subject distance D1 can be calculated without using a 3D camera.

Further, in the second embodiment, by configuring as described below, further effects as below are obtained.

That is, in the second embodiment, as described above, the optical imaging control unit 32 (control unit) calculates the imaging unit-body surface distance D5 between the optical imaging unit 31 and the body surface of the subject 101 in the optical axis direction of the irradiation field lamp 33 (light source unit), based on coordinates of the predetermined position 33c in the irradiation field region 33a appearing on the body surface of the subject 101 in the optical image 70, coordinates of the imaging unit coordinate system centered on the optical imaging unit 31, and a relational expression for converting coordinates of the imaging unit coordinate system into coordinates of the image coordinate system indicating coordinates in the optical image 70. Then, the optical imaging control unit 32 calculates the X-ray tube-subject distance D1 based on the calculated imaging unit-body surface distance D5 and the X-ray tube-imaging unit distance D6 between the X-ray tube 11 and the optical imaging unit 31 in the optical axis direction of the irradiation field lamp 33. With such a configuration, it is possible to geometrically calculate the imaging unit-body surface distance D5 based on coordinates of the predetermined position 33c in the irradiation field region 33a appearing on the body surface of the subject 101 in the optical image 70, coordinates of the imaging unit coordinate system, and a relational expression for converting coordinates of the imaging unit coordinate system into coordinates of the image coordinate system, and also possible to calculate the X-ray tube-subject distance D1 by adding the imaging unit-body surface distance D5 and the X-ray tube-imaging unit distance D6.

Further, in the second embodiment, as described above, the optical imaging control unit 32 (control unit) calculates the imaging unit-body surface distance D5 in the optical axis direction of the irradiation field lamp 33 (light source unit) based on coordinates of the predetermined position 33c in the irradiation field region 33a appearing on the body surface of the subject 101 in the optical image 70, coordinates of the imaging unit coordinate system, and the relational expression using the matrix of the internal parameters of the optical imaging unit 31. With such a configuration, coordinates of the imaging unit coordinate system and coordinates of the image coordinate system can be easily converted by the relational expression for converting coordinates of the imaging unit coordinate system into coordinates of the image coordinate system using the matrix of the internal parameters of the optical imaging unit 31, so that the imaging unit-body surface distance D5 in the optical axis direction of the irradiation field lamp 33 can be easily calculated.

Note that other effects of the second embodiment are the same as those of the first embodiment.

### [Modifications]

It should be considered that the embodiments disclosed this time are illustrative in all respects and not restrictive. The scope of the present invention is shown not by the description of the above-described embodiments but by the scope of claims, and furthermore includes all modifications (modifications) within meanings and scopes equivalent to those of the scope of claims.

For example, in the above-described first and second embodiments, the example is shown in which the optical imaging control unit 32 (232) (control unit) causes the display operation unit 41 (display unit) to display the detection unit region display 81 indicating a region where the X-ray detection unit 20 is arranged in the optical image 70, based on the calculated X-ray tube-subject distance D1, but the present invention is not limited to this. In the present invention, the control unit may not cause the display unit to display a detection unit region display indicating a region where an X-ray detection unit is arranged in an optical image, based on a calculated X-ray tube-subject distance.

Also, in the above-described first and second embodiments, the example is shown in which the optical imaging control unit 32 (232) (control unit) causes the display operation unit 41 to display the calculated X-ray tube-subject distance D1, but the present invention is not limited to this. In the present invention, the control unit may not cause a display unit to display a calculated X-ray tube-subject distance.

Also, in the above-described first and second embodiments, the example is shown in which the optical axis of the optical imaging unit 31 is parallel to the optical axis of the irradiation field lamp 33 (light source unit), but the present invention is not limited to this. In the present invention, an optical axis of an optical imaging unit may not be parallel to an optical axis of a light source unit. In that case, when calculating an X-ray tube-subject distance, it is necessary to consider that the optical axis of the optical imaging unit is inclined with respect to the optical axis of the irradiation field lamp. For example, in the case of the first embodiment, when calculating coordinates of a predetermined position of an X-ray detection unit in an optical image, it is considered that the optical axis of the optical imaging unit is inclined with respect to the optical axis of the irradiation field lamp. Further, in the case of the second embodiment, coordinates of an imaging unit coordinate system used for the above equation (1) takes into consideration that the optical axis of the optical imaging unit is inclined with respect to the optical axis of the irradiation field lamp.

Also, in the above-described first and second embodiments, the example is shown in which the optical imaging control unit 32 (232) (control unit) calculates the X-ray tube-subject distance D1 based on coordinates of a center as the predetermined position 33c in the irradiation field region 33a appearing on the body surface of the subject 101 in the optical image 70, but the present invention is not limited to this. In the present invention, a control unit may use coordinates other than a center as coordinates of a predetermined position in an irradiation field region appearing on a body surface of a subject in an optical image. In that case, coordinates of any place in the irradiation field region appearing on the body surface of the subject in the optical image may be used as long as a position can be recognized.

Also, in the above-described first embodiment, the example is shown in which the optical imaging control unit 232 (control unit) calculates the imaging unit-body surface distance D5 in the optical axis direction of the irradiation field lamp 33 (light source unit) based on coordinates of the predetermined position 33c in the irradiation field region 33a appearing on the body surface of the subject 101 in the optical image 70, coordinates of the imaging unit coordinate system, and the relational expression using the matrix of the internal parameters of the optical imaging unit 31, but the present invention is not limited to this. In the present invention, a control unit may calculate an imaging unit-body surface distance in an optical axis direction of a light source unit based on coordinates of a predetermined position in an irradiation field region appearing on a body surface of a subject in an optical image, coordinates of an imaging unit coordinate system, and a relational expression for converting coordinates of the imaging unit coordinate system into coordinates of an optical image coordinate system other than a relational expression using a matrix of internal parameters of an optical imaging unit.

Also, in the above-described first embodiment, the example is shown in which the optical imaging control unit 32 (control unit) calculates the detection unit-body surface distance D3 in the optical axis direction of the irradiation field lamp 33 based on the angle θ formed by the optical axis of the irradiation field lamp 33 (light source unit) and the straight line connecting the optical imaging unit 31 and the center 20b of the X-ray detection unit 20, in addition to the coordinate difference D2, but the present invention is not limited to this. In the present invention, a control unit may calculate a detection unit-body surface distance in an optical axis direction of a light source unit based on information recognizable by an optical imaging control unit such as relative positional relationships of respective parts of an X-ray imaging apparatus other than an angle formed by an optical axis of the light source unit and a straight line connecting an optical imaging unit and a center of an X-ray detection unit, in addition to a coordinate difference.

Also, in the above-described first embodiment, the example is shown in which the optical imaging control unit 32 (control unit) calculates the detection unit-body surface distance D3 between the X-ray detection unit 20 and the body surface of the subject 101 in the optical axis direction of the irradiation field lamp 33 (light source unit) based on the coordinate difference D2 between coordinates of the predetermined position 20a of the X-ray detection unit 20 in the optical image 70 and coordinates of the predetermined position 33c in the irradiation field region 33a appearing on the body surface of the subject 101 in the optical image 70, and also calculates the X-ray tube-subject distance D1 based on the calculated detection unit-body surface distance D3 and the X-ray tube-detection unit distance D4 between the X-ray tube 11 and the X-ray detection unit 20, and in the above-described second embodiment, the example is shown in which the optical imaging control unit (232) (control unit) calculates the imaging unit-body surface distance D5 between the optical imaging unit 31 and the body surface of the subject 101 in the optical axis direction of the irradiation field lamp 33 based on coordinates of the predetermined position 33c in the irradiation field region 33a appearing on the body surface of the subject 101 in the optical image 70, coordinates of the imaging unit coordinate system centered on the optical imaging unit 31, and a relational expression for converting coordinates of the imaging unit coordinate system into coordinates of the image coordinate system indicating coordinates in the optical image 70, and the optical imaging control unit 32 calculates the X-ray tube-subject distance D1 based on the calculated imaging unit-body surface distance D5 and the X-ray tube-imaging unit distance D6 between the X-ray tube 11 and the optical imaging unit 31 in the optical axis direction of the irradiation field lamp 33, but the present invention is not limited to this. In the present invention, a control unit may be configured to be capable of calculating an X-ray tube-subject distance by the method of the above-described first embodiment, and also be capable of calculating an X-ray tube-subject distance by the method of the above-described second embodiment.

Also, in the above-described first and second embodiments, the example is shown in which the imaging unit 30 composed of the optical imaging unit 31 and the optical imaging control unit 32 (control unit) is arranged in the holding unit 40 holding the X-ray irradiation unit 10, but the present invention is not limited to this. In the present invention, an optical imaging unit and a control unit may be arranged at positions separated from each other. Moreover, an optical imaging unit may be arranged at a position different from a holding unit, such as a ceiling or wall surface of an imaging room. Moreover, a control unit may be arranged at a position different from a holding unit. Further, an imaging unit may be configured as a retrofit unit for an X-ray imaging apparatus.

Also, in the above-described first and second embodiments, the example is shown in which the detection unit region display 81 has the cross-shaped center display 82 and the four region displays 83 indicating four corners, and the irradiation field region display 84 has the region display 85 which is a rectangular frame line and the pair of dotted-line displays 86, but the present invention is not limited to this. In the present invention, a detection unit region display and an irradiation field region display may be displayed in a mode different from those in the above embodiments. For example, either a detection unit region display or an irradiation field region display may not include a display indicating a center of a region. Further, in a detection unit region display, a range of a region may be indicated by a rectangular frame line. In an irradiation field region display, a pair of dotted-line displays may not be shown, and only a rectangular frame line may be shown, or a range of a rectangular region may be shown only by displaying four corners. At least one of a detection unit region display and an irradiation field region display may be displayed transparently, or may be displayed blinking.

### [Aspects]

It is understood by those skilled in the art that the above-described exemplary embodiments are specific examples of the following aspects.

### (Item 1)

An X-ray imaging apparatus comprising: an X-ray irradiation unit including an X-ray tube; an X-ray detection unit configured to detect X-rays irradiated from the X-ray irradiation unit and transmitted through a subject; a light source unit configured to irradiate visible light to an irradiation field region indicating a region to be irradiated with X-rays by the X-ray irradiation unit; an optical imaging unit having an optical axis shifted with respect to an irradiation axis of the X-rays irradiated from the X-ray irradiation unit, and configured to capture an optical image of the subject; and a control unit configured to calculate an X-ray tube-subject distance between the X-ray tube and the subject based on coordinates of a predetermined position in the irradiation field region appearing on a body surface of the subject in the optical image captured by the optical imaging unit.

### (Item 2)

The X-ray imaging apparatus according to item 1, wherein the optical imaging unit includes a monocular camera, and the control unit calculates the X-ray tube-subject distance based on coordinates of the predetermined position in the irradiation field region appearing on the body surface of the subject in the optical image captured by the monocular camera.

### (Item 3)

The X-ray imaging apparatus according to item 1 or 2, wherein the control unit calculates a detection unit-body surface distance between the X-ray detection unit and the body surface of the subject in an optical axis direction of the light source unit based on a coordinate difference between coordinates of a predetermined position of the X-ray detection unit in the optical image and coordinates of the predetermined position in the irradiation field region appearing on the body surface of the subject in the optical image, and calculates the X-ray tube-subject distance based on the calculated detection unit-body surface distance and an X-ray tube-detection unit distance between the X-ray tube and the X-ray detection unit.

### (Item 4)

The X-ray imaging apparatus according to item 3, wherein the control unit calculates the detection unit-body surface distance in an optical axis direction of the X-ray tube based on an angle formed by an optical axis of the light source unit and a straight line connecting the optical imaging unit and a center of the X-ray detection unit, in addition to the coordinate difference.

### (Item 5)

The X-ray imaging apparatus according to item 1 or 2, wherein the control unit calculates an imaging unit-body surface distance between the optical imaging unit and the body surface of the subject in an optical axis direction of the light source unit based on coordinates of the predetermined position in the irradiation field region appearing on the body surface of the subject in the optical image, coordinates of an imaging unit coordinate system centered on the optical imaging unit, and a relational expression for converting coordinates of the imaging unit coordinate system into coordinates of an image coordinate system indicating coordinates in the optical image, and calculates the X-ray tube-subject distance based on the calculated imaging unit-body surface distance and an X-ray tube-imaging unit distance between the X-ray tube and the optical imaging unit in the optical axis direction of the light source unit.

### (Item 6)

The X-ray imaging apparatus according to item 5, wherein the control unit calculates the imaging unit-body surface distance in the optical axis direction of the light source unit based on coordinates of the predetermined position in the irradiation field region appearing on the body surface of the subject in the optical image, coordinates of the imaging unit coordinate system, and the relational expression using a matrix of internal parameters of the optical imaging unit.

### (Item 7)

The X-ray imaging apparatus according to any one of items 1 to 6, wherein the control unit calculates the X-ray tube-subject distance based on coordinates of a center as the predetermined position in the irradiation field region appearing on the body surface of the subject in the optical image.

### (Item 8)

The X-ray imaging apparatus according to any one of items 1 to 7, wherein an optical axis of the optical imaging unit is parallel to an optical axis of the light source unit.

### (Item 9)

The X-ray imaging apparatus according to any one of items 1 to 8, further comprising a display unit, wherein the control unit causes the display unit to display the calculated X-ray tube-subject distance.

### (Item 10)

The X-ray imaging apparatus according to any one of items 1 to 9, wherein the control unit causes the display unit to display a detection unit region display indicating a region where the X-ray detection unit is arranged in the optical image, based on the calculated X-ray tube-subject distance.

### Reference Signs List

10 X-ray irradiation unit
11 X-ray tube
20 X-ray detection unit
20a Predetermined position (of X-ray detection unit in optical image)
20b Center of X-ray detection unit
31 Optical imaging unit
31b Monocular camera
31c Optical axis (of optical imaging unit)
32, 232 Optical imaging control unit (Control unit)
33 Irradiation field lamp (Light source unit)
33a Irradiation field region
33b Optical axis (of light source unit)
33c Predetermined position (in irradiation field region appearing on body surface of
subject in optical image)
41 Display operation unit (Display unit)
70 Optical image
81 Detection unit region display
100, 200 X-ray imaging apparatus
101 Subject
D1 X-ray tube-subject distance
D2 Coordinate difference
D3 Detection unit-body surface distance
D4 X-ray tube-detection unit distance
D5 Imaging unit-body surface distance
D6 X-ray tube-imaging unit distance
θ Angle (formed by an optical axis of light source unit and a straight line connecting optical imaging unit and a center of X-ray detection unit)

## Claims

1. An X-ray imaging apparatus comprising: an X-ray irradiation unit including an X-ray tube; an X-ray detection unit configured to detect X-rays irradiated from the X-ray irradiation unit and transmitted through a subject; a light source unit configured to irradiate visible light to an irradiation field region indicating a region to be irradiated with X-rays by the X-ray irradiation unit; an optical imaging unit having an optical axis shifted with respect to an irradiation axis of the X-rays irradiated from the X-ray irradiation unit, and configured to capture an optical image of the subject; and a control unit configured to calculate an X-ray tube-subject distance between the X-ray tube and the subject based on coordinates of a predetermined position in the irradiation field region appearing on a body surface of the subject in the optical image captured by the optical imaging unit.

2. The X-ray imaging apparatus according to claim 1, wherein the optical imaging unit includes a monocular camera, and the control unit calculates the X-ray tube-subject distance based on coordinates of the predetermined position in the irradiation field region appearing on the body surface of the subject in the optical image captured by the monocular camera.

3. The X-ray imaging apparatus according to claim 1, wherein the control unit calculates a detection unit-body surface distance between the X-ray detection unit and the body surface of the subject in an optical axis direction of the light source unit based on a coordinate difference between coordinates of a predetermined position of the X-ray detection unit in the optical image and coordinates of the predetermined position in the irradiation field region appearing on the body surface of the subject in the optical image, and calculates the X-ray tube-subject distance based on the calculated detection unit-body surface distance and an X-ray tube-detection unit distance between the X-ray tube and the X-ray detection unit.

4. The X-ray imaging apparatus according to claim 3, wherein the control unit calculates the detection unit-body surface distance in an optical axis direction of the light source unit based on an angle formed by an optical axis of the light source unit and a straight line connecting the optical imaging unit and a center of the X-ray detection unit, in addition to the coordinate difference.

5. The X-ray imaging apparatus according to claim 1, wherein the control unit calculates an imaging unit-body surface distance between the optical imaging unit and the body surface of the subject in an optical axis direction of the light source unit based on coordinates of the predetermined position in the irradiation field region appearing on the body surface of the subject in the optical image, coordinates of an imaging unit coordinate system centered on the optical imaging unit, and a relational expression for converting coordinates of the imaging unit coordinate system into coordinates of an image coordinate system indicating coordinates in the optical image, and calculates the X-ray tube-subject distance based on the calculated imaging unit-body surface distance and an X-ray tube-imaging unit distance between the X-ray tube and the optical imaging unit in the optical axis direction of the light source unit.

6. The X-ray imaging apparatus according to claim 5, wherein the control unit calculates the imaging unit-body surface distance in an optical axis direction of the X-ray tube based on coordinates of the predetermined position in the irradiation field region appearing on the body surface of the subject in the optical image, coordinates of the imaging unit coordinate system, and the relational expression using a matrix of internal parameters of the optical imaging unit.

7. The X-ray imaging apparatus according to claim 1, wherein the control unit calculates the X-ray tube-subject distance based on coordinates of a center as the predetermined position in the irradiation field region appearing on the body surface of the subject in the optical image.

8. The X-ray imaging apparatus according to claim 1, wherein an optical axis of the optical imaging unit is parallel to an optical axis of the light source unit.

9. The X-ray imaging apparatus according to claim 1, further comprising a display unit, wherein the control unit causes the display unit to display the calculated X-ray tube-subject distance.

10. The X-ray imaging apparatus according to claim 9, wherein the control unit causes the display unit to display a detection unit region display indicating a region where the X-ray detection unit is arranged in the optical image, based on the calculated X-ray tube-subject distance.
